# EUROPEAN PATENT APPLICATION

(11) **EP 4 201 922 A1**
(43) Date of publication of application: **28.06.2023**
(21) Application number: 22214412.3
(22) Date of filing: 19.12.2022
(51) Int. Cl.: C07D 207/26

(54) **METHOD FOR PURIFYING WASTED SOLUTION COMPRISING N-METHYL-2-PYRROLIDONE**

(30) Priority: 22.12.2021 KR 20210184747
(71) Applicant: JAEWON INDUSTRIAL CO., LTD, Jeollanam-do 59618 (KR)
(72) Inventor: SHIM, Sung Won, 59618 Yeosu-si (KR); SON, Byung Ki, 34119 Yuseong-gu (KR); CHOI, Hwan, 59723 Yeosu-si (KR); SHIN, Youn Soo, 59692 Yeosu-si (KR); CHOI, Hwa Yeong, 59666 Yeosu-si (KR); CHOI, Tae Gi, 59640 Yeosu-si (KR); CHOO, Yeong Su, 59674 Yeosu-si (KR)
(74) Representative: Vossius & Partner Patentanwälte Rechtsanwälte mbB

(57) **Abstract**

Provided is a method for purifying waste N-methyl-2-pyrrolidone, the method comprising the steps of reacting the waste N-methyl-2-pyrrolidone containing an amine compound with an acid anhydride; converting the amine compound into an amide compound; and removing the amide compound by distillation, thereby manufacturing high-purity N-methyl-2-pyrrolidone that can be reused.

## Description

### TECHNICAL FIELD

The present invention relates to a method for purifying a waste solution containing N-methyl-2-pyrrolidone (hereinafter, referred to as waste NMP) and more particularly, to a method for purifying a waste solution containing N-methyl-2-pyrrolidone in which an amine compound contained in waste NMP is reacted with an acid anhydride and converted into an amide compound, which is then removed to obtain high-purity N-methyl-2-pyrrolidone.

### BACKGROUND

N-methyl-2-pyrrolidone (NMP) is an organic solvent that has low viscosity, is colorless and non-toxic, and has excellent heat resistance. Since NMP is a chemically stable and highly polar solvent, it is very useful for various chemical reactions that require an inert medium.

NMP is a product in which as environmental regulations are becoming stricter, the demand is growing as an eco-friendly non-toxic product in the field of polymer polymerization and processing solvents, paint manufacturing solvents, metal surface cleaners, pharmaceutical synthesis and purification solvents, processing solvents for semiconductors and electronic materials, lithium battery manufacturing solvents, and the like. Particularly, NMP has a solvent which has a hydrophilic group and a hydrophobic group in the chemical structure and is excellent in performance for dissolving or diluting other materials, and thus, is widely used in the field of electronic materials.

When waste NMP generated in the electronics industry is recovered and reused in the process, not only the economic effect but also the effect of reducing the emission of environmental pollutants is significant. Therefore, methods for recovering used waste NMP, purifying it into high-purity NMP, and then reusing it are being actively studied.

If the amine compound that has flowed into NMP during the manufacturing process of displays, semiconductors, and secondary batteries is not completely removed, defects can be induced in the display or semiconductor cleaning process, and in the secondary battery process, defects can be induced in the stage prior to dispersing a positive electrode material. The amine compound present in waste NMP can be purified by a distillation process, but it does not have a large boiling point difference from NMP, or it is not easy to remove a material that performs azeotropic distillation.

Therefore, in order to obtain high-purity NMP, there is a need to study a method for purifying waste NMP that can effectively remove amine compounds contained in waste NMP.

### [Prior Art Literature]

### [Patent Literature]

(Patent Literature 1) Korean Publication Patent No. 10-2015-008506 (published on July 22, 2015)
(Patent Literature 2) Korean Publication Patent No. 10-2018-0069284 (published on June 25, 2018)

### DETAILED DESCRIPTION OF THE INVENTION

### Technical Problem

It is an object of the present invention to provide a method for purifying a waste solution containing N-methyl-2-pyrrolidone in which an amine compound present in NMP raw material itself or an amine compound flown into waste NMP used in display process, semiconductor process, secondary battery manufacturing process, and the like is converted into an amide compound, which can be effectively removed to obtain high-purity N-methyl-2-pyrrolidone.

### Technical Solution

In one aspect of the present invention, there is provided a method for purifying a waste solution containing N-methyl-2-pyrrolidone, the method comprising: reacting waste N-methyl-2-pyrrolidone containing an amine compound with an acid anhydride, converting the amine compound into an amide compound and removing the amide compound by distillation.

In one embodiment, as shown in the following Reaction Scheme, waste N-methyl-2-pyrrolidone containing an amine compound represented by Formula 1 reacts with an acid anhydride represented by Formula 2 to produce an amide compound represented by Formula 3.

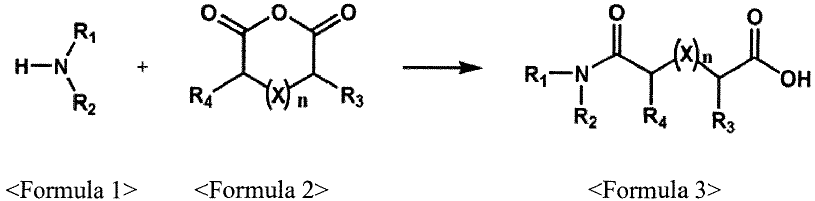

It is preferable to add a basic material in the step of converting into the amide compound and add an antioxidant in the distillation step.

Also, preferably, the weight of the acid anhydride is 2 times or more the weight of the amine compound contained in the waste N-methyl-2-pyrrolidone.

### ADVANTAGEOUS EFFECTS

According to the present invention, it is possible to effectively remove amine compounds contained in waste NMP and produce high-purity NMP that can be reused in the electronics industry.

### BRIEF DESCRIPTION OF THE DRAWINGS

Fig. 1 is a gas chromatogram of waste N-methyl-2-pyrrolidone before purification.
Fig. 2 is a gas chromatogram of purified N-methyl-2-pyrrolidone according to an embodiment of the present invention.

### DETAILED DESCRIPTION OF THE EMBODIMENTS

Hereinafter, exemplary embodiments of the present invention will be described in detail with reference to the attached drawings

Unless otherwise defined, all terms used herein are identical to a general meaning of terms understood by one of ordinary skill in the art. A term which conflicts with a general meaning thereof will be understood according to a meaning defined herein.

Throughout the specification, when a portion is referred to as "including" a certain component, it means that the portion can further include other components, without excluding the other components, unless otherwise stated.

Further, the waste N-methyl-2-pyrrolidone as used herein may be expressed as waste NMP, which does not mean a single NMP compound, but means containing impurities such as amine compounds.

Further, the amide compound as used herein refers to a compound containing 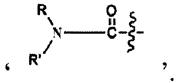 Here, R and R' are substituent groups.

In one embodiment, the waste NMP purification method according to the present invention may comprise a step of preparing waste N-methyl-2-pyrrolidone containing an amine compound, a step of reacting the waste NMP with an acid anhydride, and a step of distilling the product in a distillation column after the reaction, and further comprise a step of filtering the purified NMP by distillation.

The purification method will be described for each step in more detail below.

First, waste N-methyl-2-pyrrolidone containing an amine compound is prepared. The amine compound may be present in the raw material or may be flown into the semiconductor manufacturing process. Since such an amine compound may cause defects in the semiconductor manufacturing process, the amine compound must be removed if the waste NMP is desired to be reused.

The amine compound is usually contained in waste NMP in an amount of 100-10,000 ppm. According to the present invention, it is possible to effectively remove primary amines and secondary amines contained in waste NMP.

For example, the amine compound contained in waste NMP may be one or more selected from the group consisting of methylamine, dimethylamine, monomethanolamine, monoethanolamine, diethanol amine, 3-amino-1-propanol, 2-amino-1-propanol, methyl monoethanolamine, 4-amino-1-butanol, 5-amino-1-pentanol, ethyl monoethanol amine, propyl monoethanolamine, butyl monoethanolamine, 2-amino-2-methyl-propanol, 2-amino-2-ethyl-1-propanol, 2-amino-2-methyl-1,3-propanediol, and piperazine.

Next, the waste NMP is reacted with an acid anhydride, so that the amine compound contained in the waste NMP is converted into an amide compound.

The amide compound is a high boiling point compound, and has a large boiling point difference from NMP. Therefore, after converting the amine compounds contained in waste NMP into amide compounds and then by removing the amide compound, the amine compound contained in waste NMP can be effectively removed.

The acid anhydride may be one or more selected from the group consisting of maleic anhydride, bromomaleic anhydride, phenylmaleic anhydride, succinic anhydride, methylsuccinic anhydride, phenylsuccinic anhydride, phthalic anhydride, 3-hydrophthalic anhydride, tetrahydrophthalic anhydride, 3-nitrophthalic anhydride, 3-fluorophthalic anhydride, hexahydrophthalic anhydride, 4-tert-butylphthalic anhydride, pyromellitic dianhydride, itaconic anhydride, citraconic anhydride, 3-methylglutaric anhydride, 3,3-tetramethyleneglutaric anhydride, 2-phenylglutaric anhydride and 1-cyclopentene-1,2-dicarboxylic anhydride.

The acid anhydride is preferably in a cyclic form. This is because cyclic anhydrides do not produce other by-products when reacted with waste NMP.

At this time, the weight of the acid anhydride is 2 times or more, preferably 2 to 4 times the weight of the amine compound contained in the waste NMP. If the weight of acid anhydride is less than 2 times the weight of the amine, all of the amine compounds cannot be converted into amide compounds, which causes a problem that unreacted amine compounds remain in purified NMP, and when the acid anhydride is used in a too much amount, it is uneconomical and causes problems such as the need to remove the remaining acid anhydride.

Preferably, a basic material may be further added in the step of reacting the waste NMP with the acid anhydride. Since the waste NMP may contain various unknown impurities, especially acid materials, the acid value is lowered by adding a basic material, and an environment can be created in which the waste NMP can react better with the acid anhydride.

At this time, the basic material may be one or more selected from the group consisting of KOH, NaOH, Ba(OH)₂, CsOH, Sr(OH)₂, Ca(OH)₂, LiOH, RbOH, n-BuLi, NaH, Na₂CO₃, NaHCO₃, K₂CO₃, KHCO₃, CaCO₃ and CaHCO₂.

When converting the amine and acid anhydride into a high-boiling amide compound in the presence of a basic material, the reaction is performed preferably at a temperature of 100-150°C, preferably 110-130°C. When the reaction temperature is less than 100°C, the reaction may proceed slowly, and when the reaction is performed at a temperature higher than 150°C, NMP may be oxidized and thermally decomposed.

In one embodiment, the amine compound and the acid anhydride contained in the waste NMP can be reacted according to the following Reaction Scheme 1 to form an amide compound. Here, the amine compound is represented by Formula 1, the acid anhydride is represented by Formula 2, and the amide compound is represented by Formula 3.

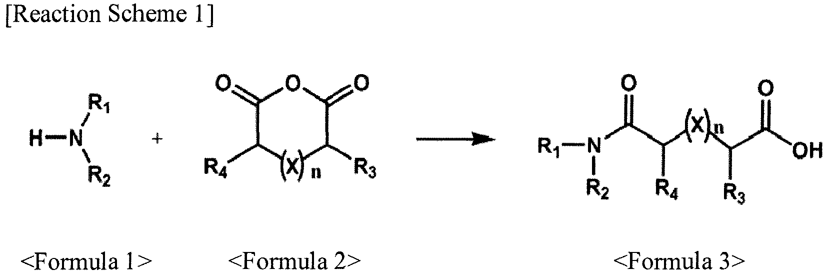

In Formulas 1 to 3, each symbol may be defined as follows.

R₁ and R₂ are each independently selected from the group consisting of hydrogen, a C₁-C₂₀ alkyl group, a C₆-C₃₀ aryl group, a C₆-C₃₀ cycloalkyl group, a C₁-C₂₀ alkoxy group, a C₆-C₃₀ aryloxy group, -OH and a C₁-C₂₀ alkyl alcohol group, with the proviso that the case where both R₁ and R₂ are hydrogen is excluded, and R₁ and R₂ may be bonded to each other to form a ring.

When one of R₁ and R₂ is hydrogen, compound of Formula 1 corresponds to a primary amine compound, and when both R₁ and R₂ are substituents other than hydrogen, compound of Formula 1corresponds to a secondary amine compound.

When R₁ and R₂ are linked to each other to form a ring, a heterocycle containing at least one N is formed. Here, the heterocycle may be a C₂-C₂₀, a C₂-C₁₀, a C₂-C₉, a C₂-C₈, a C₂-C₇, a C₂-C₅, a C₂-C₄, a C₂-C₃, a C₂, a C₃, a C₄, a C₅, a C₆, a C₇, a C₈, a C₉ or a C₁₀ heterocycle, for example, triazine, pyrimidine, pyridine, piperazine, quinoline, quinazoline, etc.

H of the alkyl group, the aryl group, the cycloalkyl group, the alkoxy group, the aryloxy group and the alkyl alcohol group, etc. may be replaced with a C₁-C₂₀ alkyl group, an alcohol group, or a C₁-C₂₀ alkyl alcohol group.
n is an integer of 0 or 1, when n is 0, X is a single or double bond, and when n is 1, X is C(R')(R").

For example, when n is 0, the acid anhydride may be represented by the following Formula 2-1 or 2-2, and when n is 1, the acid anhydride may be represented by the following Formula 2-3.

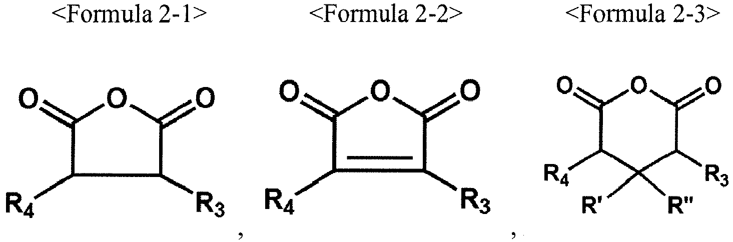

Therefore, Reaction Scheme 1may be represented by the following Reaction Scheme 2 when the acid anhydride is Formula 2-1 in Reaction Scheme 1, it may be represented by the following Reaction Scheme 3 when the acid anhydride is Formula 2-2, and it may be represented by Scheme 4 when the acid anhydride may be represented by Formula 2-3.

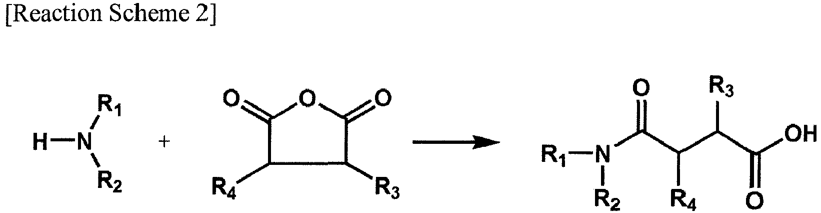

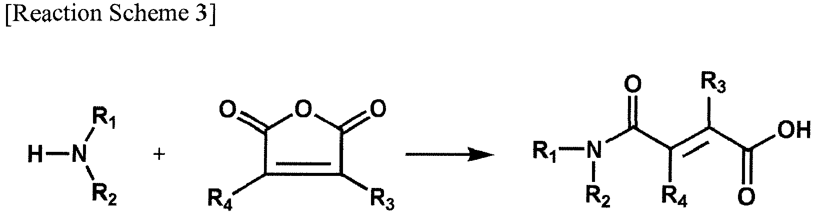

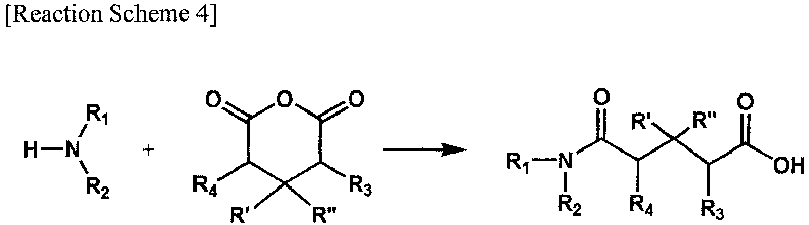

In Formulas 2, 3, 2-1, 2-2, and 2-3, R₃ and R₄ are each independently selected from the group consisting of hydrogen, halogen, a C₁-C₂₀ alkyl group, a C₂-C₂₀ alkenyl group, a C₆-C₃₀ aryl group, a C₆-C₃₀ cycloalkyl group, -OH and a C₁-C₂₀ alkyl alcohol group, and in Formulas 2-1 and 2-2, R₃ and R₄ may be linked to each other to form a ring. At this time, the ring may be substituted with one or more R₅, wherein R₅s are the same or different from each other.

R₅ is selected from the group consisting of hydrogen, halogen, nitro group, a C₁-C₂₀ alkyl group, a C₂-C₂₀ alkenyl group, a C₆-C₃₀ aryl group, a C₆-C₃₀ cycloalkyl group, -OH and a C₁-C₂₀ alkyl alcohol group.

The ring formed by R₃ and R₄ may be a C₆-C₃₀ aromatic hydrocarbon ring, a C₆-C₃₀ aliphatic ring, or a C₂-C₃₀ heterocyclic ring, for example, benzene, naphthalene, cyclopentane, cyclohexane, phthalic anhydride, and the like.

H in the alkyl group, the alkenyl group, the aryl group, the cycloalkyl group, the alkyl alcohol group, etc. may be replaced with a C₁-C₂₀ alkyl group, an alcohol group, or a C₁-C₂₀ alkyl alcohol group.

In Formula 2-1, compound of Formula 2-1 corresponds to succinic anhydride when both R₃ and R₄ are hydrogen, it corresponds to phthalic anhydride when R₃ and R₄ are linked to each other to form a benzene ring, it corresponds to hexahydrophthalic anhydride when R₃ and R₄ are linked to each other to form a cyclohexane ring, and it corresponds to pyromellitic dianhydride when R₃ and R₄ are linked to each other to form a phthalic anhydride ring.

In Formula 2-2, compound of Formula 2-2 corresponds to maleic anhydride when both R₃ and R₄ are hydrogen, it corresponds to phthalic anhydride when R₃ and R₄ are linked to each other to form a benzene ring, and it corresponds to pyromellitic dianhydride when R₃ and R₄ are linked to each other to form a phthalic anhydride ring.

R' and R" are each independently selected from the group consisting of hydrogen, a C₁-C₂₀ alkyl group, a C₂-C₂₀ alkenyl group, a C₆-C₃₀ aryl group and a C₆-C₃₀ cycloalkyl group, R' and R" may be linked to each other to form a ring. The ring by R' and R" may be a C₆-C₃₀ aromatic hydrocarbon ring, a C₆-C₃₀ aliphatic ring, or a C₂-C₃₀ heterocycle, for example, benzene, naphthalene, cyclopentane, cyclohexane, etc. When R' and R" are linked to each other to form a ring, a spiro-compound can be formed.

In Formula 2-3, compound of Formula 2-3 corresponds to 3-methylglutaric anhydride when R₃, R₄ and R' are all hydrogen and R" is a methyl group, it corresponds to 3,3-tetramethyleneglutaric anhydride when both R₃ and R₄ are hydrogen, and R' and R" are linked to each other to form cyclopentane.

When at least one of R₁ to R₅ is an alkyl group, the alkyl group may be a C₁-C₂₀ alkyl group, a C₁-C₁₀ alkyl group, a C₁-C₅ alkyl group, a C₁-C₄ alkyl group, a C₁, a C₂, a C₃, a C₄ or a C₅ alkyl group, specifically, a methyl group, an ethyl group, a propyl group, a butyl group, a t-butyl group, and the like.

When at least one of R₁ to R₅ is an alkyl alcohol group, the alkyl alcohol group is a C₁-C₂₀ alkyl alcohol group, a C₁-C₁₀ alkyl alcohol group, a C₁-C₅ alkyl alcohol group, a C₁-C₄ alkyl alcohol group, a C₁, a C₂, a C₃, a C₄ or a C₅ alkyl alcohol group, specifically, a methyl alcohol group, an ethyl alcohol group, a propyl alcohol group, a butyl alcohol group, a pentyl alcohol group, and the like.

When at least one of R₁ to R₅ is an aryl group, the aryl group is a C₆-C₃₀ aryl group, C₆-C₂₀ aryl group, C₆-C₁₈ aryl group, C₆-C₁₂ aryl group, C₆-C₁₀ aryl group, a C₆, a C₁₀ or a C₁₂ aryl group, specifically, phenyl, biphenyl, naphthyl, phenanthrenyl, and the like.

When at least one of R₃ to R₅ is an alkenyl group, the alkenyl group is a C₁-C₂₀ alkenyl group, a C₁-C₁₀ alkenyl group, a C₁-C₅ alkenyl group, a C₁-C₄ alkenyl group, a C₁, a C₂, a C₃, a C₄ or a C₅ alkenyl group, specifically, a methylene group, an ethylidenyl group, a propylideyl group, and the like.

Next, waste NMP containing amide compound produced by Reaction Scheme 1 is distilled to remove the amide compound having a high boiling point. In the distillation step, unreacted acid anhydrides and other impurities in the reactants can also be removed.

Since NMP can be oxidized during distillation to produce NMP PEROXIDE, NMS (N-methyl succinimide), and the like, antioxidants can be added to remove these by-products.

As the antioxidant, at least one of a primary antioxidant, phenolic antioxidant, and a secondary antioxidant, phosphite antioxidant, may be used.

As the phenolic antioxidant, BHT (butylated hydroxytoluene), MeHQ (hydroquinone monomethyl ether), TBC (4-tert-butylcatechol), HQ (hydroquinone), Songnox 1010, Songnox 1076, Songnox 1135, Songnox 2450, or Songnox 1035 is typically used.

As the phosphite antioxidant, tris(2,4-di-tert-butylphenyl)phosphite, bis(2,4-di-tert-butylphenyl)pentaerythritol diphosphate and the like are typically used.

The primary antioxidant and the secondary antioxidant may be added in an amount of 10 to 10,000 ppm, preferably 100 to 2,000 ppm, based on the total weight of NMP.

In the distillation step, the amide compound having a high boiling point can be discharged to the bottom of the distillation column and removed, and purified NMP can be obtained at the upper part of the distillation column.

The distillation column can use a tray column or a packing column, and the purification by distillation can be performed at a reboiler temperature of 80 to 130°C, preferably at 100 to 120°C.

Finally, in order to remove impurities such as metal and particles contained in the purified NMP obtained from the upper part of the distillation column, a filtration step may be performed. For example, the purified NMP is passed through an ion filter to remove metal. Subsequently, it continuously passes through a particle filter to remove particles, thereby finally manufacturing a high-purity NMP product.

Hereinafter, examples of a method for purifying a mixed solution of waste NMP according to an embodiment of the present invention will be described. These examples are for illustrative purposes only, and the scope of the invention is not limited thereby.

### Examples and Comparative Examples

### [Example 1]

100 parts by weight of waste NMP containing 0.063 parts by weight of butylmonoethanolamine, 0.18 parts by weight of phthalic anhydride, and 0.01 parts by weight of NaOH were placed into a reactor, and the mixture was stirred at 130°C for 30 minutes. When the reaction was completed, the reactants were distilled in a 30-stage tray column in which the bottom temperature of the distillation column was maintained at 100-120° C and the vacuum degree at - 720 mmHg. At this time, 0.01 parts by weight of Songnox 1010 antioxidant was added to the distillation column. The amide compound, which is a high boiling point compound, was removed by distillation to the bottom of the column, and unreacted phthalic anhydride was removed to obtain high-purity purified NMP.

### <Example 2>

Purified NMP was obtained in the same manner as in Example 1, except that 0.14 g parts by weight of phthalic anhydride was used.

### <Example 3>

Purified NMP was obtained in the same manner as in Example 1, except that waste NMP containing 0.102 parts by weight of butyl monoethanolamine, 0.30 parts by weight of succinic anhydride instead of phthalic anhydride, and 0.01 part by weight of KOH instead of NaOH were used.

### <Example 4>

Purified NMP was obtained in the same manner as in Example 3, except that 0.24 g parts by weight of succinic anhydride was used.

### <Example 5>

Purified NMP was obtained in the same manner as in Example 1, except that waste NMP containing 0.066 parts by weight of diethanolamine was used instead of butyl monoethanolamine.

### <Example 6>

Purified NMP was obtained in the same manner as in Example 5, except that 0.14 g parts by weight of phthalic anhydride was used.

### <Example 7>

Purified NMP was obtained in the same manner as in Example 5, except that waste NMP containing 0.118 parts by weight of diethanolamine, 0.30 parts by weight of succinic anhydride instead of phthalic anhydride, and 0.01 part by weight of KOH instead of NaOH were used.

### <Example 8>

Purified NMP was obtained in the same manner as in Example 7, except that 0.24 parts by weight of succinic anhydride was used.

### <Comparative Example 1>

Purified NMP was obtained in the same manner as in Example 1, except that 0.10 parts by weight of phthalic anhydride was used.

### <Comparative Example 2>

Purified NMP was obtained in the same manner as in Example 3, except that 0.15 parts by weight of succinic anhydride was used.

### <Comparative Example 3>

Purified NMP was obtained in the same manner as in Example 5, except that 0.10 parts by weight of phthalic anhydride was used.

### <Comparative Example 4>

Purified NMP was obtained in the same manner as in Example 7, except that 0.15 parts by weight of succinic anhydride was used.

The amount of the reactant, amount of amine contained in the purified NMP, and purity (%) of the purified NMP of Comparative Examples 1 to 4 and Examples 1 to 8 are shown in Table 1 below.

**[Table 1]**

| | amount of amine | type of amine | amount of acid anhydride | type of acid anhydride | base | acid anhydride/ amine | residual amine | purity |
|---|---|---|---|---|---|---|---|---|
| Example 1 | 0.063 | butyl monoethanolamine | 0.18 | phthalic anhydride | NaOH | 2.86 | 0 | 99.98 |
| Example 2 | 0.063 | butyl monoethanolamine | 0.14 | phthalic anhydride | NaOH | 2.22 | 0 | 99.98 |
| Example 3 | 0.102 | butyl monoethanolamine | 0.30 | anhydrous succinic acid | KOH | 2.94 | 0 | 99.98 |
| Example 4 | 0.102 | butyl monoethanolamine | 0.24 | anhydrous succinic acid | KOH | 2.35 | 0 | 99.98 |
| Example 5 | 0.066 | diethanolamine | 0.18 | phthalic anhydride | NaOH | 2.73 | 0 | 99.98 |
| Example 6 | 0.066 | diethanolamine | 0.14 | phthalic anhydride | NaOH | 2.12 | 0 | 99.98 |
| Example 7 | 0.118 | diethanolamine | 0.30 | anhydrous succinic acid | KOH | 2.54 | 0 | 99.98 |
| Example 8 | 0.118 | diethanolamine | 0.24 | anhydrous succinic acid | KOH | 2.03 | 0 | 99.98 |
| Comparative Example 1 | 0.063 | butyl monoethanolamine | 0.10 | phthalic anhydride | NaOH | 1.59 | 0.0123 | 99.97 |
| Comparative Example 2 | 0.102 | butyl monoethanolamine | 0.15 | anhydrous succinic acid | KOH | 1.47 | 0.0353 | 99.94 |
| Comparative Example 3 | 0.066 | diethanolamine | 0.10 | phthalic anhydride | NaOH | 1.52 | 0.0187 | 99.96 |
| Comparative Example 4 | 0.118 | diethanolamine | 0.15 | anhydrous succinic acid | KOH | 1.27 | 0.0511 | 99.93 |

Examples 1 to 4 and Comparative Examples 1 and 2 were obtained by purifying waste NMP containing butylmonoethanolamine, which is a primary amine, and Examples 5 to 8 and Comparative Examples 3 and 4 were obtained by purifying waste NMP containing diethanolamine, which is a secondary amine.

As a result of experiment while changing the amount of amine and the amount of acid anhydride, when the weight of acid anhydride was 2 times or more the weight of amine contained in waste NMP, no residual amine was observed in the purified NMP, and the purity was 99.98%. That is, it can be seen that when the weight of the acid anhydride is 2 times or more the weight of the amine, all of the amines are converted into amide compounds, which can be removed by purification.

On the other hand, as can be seen in Comparative Examples 1 to 4, if the weight of acid anhydride is less than 2 times the weight of amine contained in the waste NMP, considering that amines are observed in the purified NMP, it can be seen that all of the amines contained in the waste NMP were not converted into amide compounds.

Therefore, in order to completely remove the amine compounds contained in waste NMP and to obtain high-purity purified NMP, it can be seen that the weight of acid anhydride should be 2 times or more the weight of the amine compound.

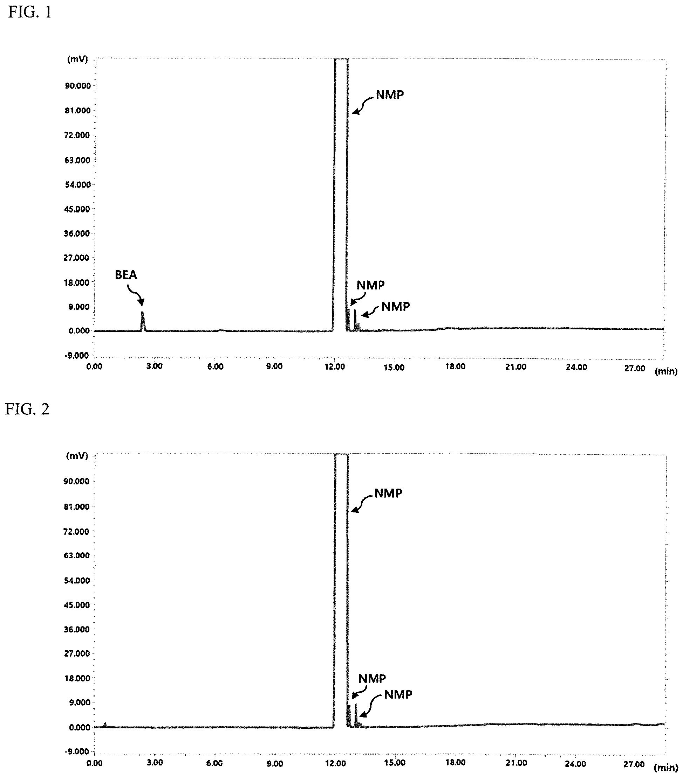

The results of analyzing the components of the raw material waste NMP used in Example 1 are shown in Table 2 below, and a gas chromatography chart is as shown in Fig. 1. In Fig. 1, only butyl monoethanolamine (denoted as BEA) and NMP among the components contained in the raw material waste NMP are indicated, and in Table 2, N-1 and N-2 also correspond to NMP. Therefore, it can be seen that the purity of the NMP before purification is 99.91 %.

**[Table 2)**

| Retention time [min] | Area [uV*sec] | Area% [%] | Component name |
|---|---|---|---|
| 2.403 | 44374 | 0.0631 | BEA |
| 2.800 | 463 | 0.0007 | UK1 |
| 3.943 | 465 | 0.0007 | UK2 |
| 4.103 | 449 | 0.0006 | UK3 |
| 6.280 | 5332 | 0.0076 | UK4 |
| 8.397 | 1259 | 0.0018 | UK5 |
| 11.193 | 289 | 0.0004 | UK6 |
| 12.593 | 70267365 | 99.8447 | NMP |
| 12.713 | 21363 | 0.0304 | N-1 |
| 13.040 | 21494 | 0.0305 | N-2 |
| 13.183 | 11364 | 0.0161 | UK7 |
| 13.470 | 900 | 0.0013 | UK8 |
| 14.206 | 612 | 0.0009 | UK9 |
| 14.553 | 506 | 0.0007 | UK10 |
| 14.870 | 405 | 0.0006 | UK11 |
| Σ | 70376638 | 100.0000 | |

| | | | |
|---|---|---|---|
| (UK: unknown component) | | | |

The results of analyzing the components of purified NMP after the purification process according to Example 1 are as shown in Table 3 below, and a gas chromatogram is as shown in Fig. 2.

**[Table 3]**

| Retention time [min] | Label | Area [uV*sec] | Hight [uV] | Area% [%] | Component name |
|---|---|---|---|---|---|
| 6.250 | BMB | 5059 | 260 | 0.0073 | UK1 |
| 6.733 | BMB | 661 | 88 | 0.0010 | UK2 |
| 8.387 | BMB | 410 | 40 | 0.0006 | UK3 |
| 12.590 | BV | 69317167 | 4049120 | 99.9204 | NMP |
| 12.710 | VB | 19909 | 7740 | 0.0287 | N-1 |
| 13.036 | BV | 21509 | 7846 | 0.0310 | N-2 |
| 13.180 | VV | 3875 | 1505 | 0.0056 | UK4 |
| 13.236 | VB | 3472 | 1305 | 0.0050 | UK5 |
| 13.466 | BB | 343 | 144 | 0.0005 | UK6 |
| Σ | | 69372406 | 4068049 | 100.0000 | |

| | | | | | |
|---|---|---|---|---|---|
| (UK: unknown component) | | | | | |

As can be seen in Table 3 and Fig. 2, it can be confirmed that butylmonoethanolamine was not observed in the purified NMP according to the present invention, and many impurities, which are other unknown components, have also been removed. As a result, it was confirmed that the purity of the purified NMP was 99.98%. In Table 3, N-1 and N-2 components also correspond to NMP.

Therefore, when purifying the waste NMP according to the present invention, the amine compound can be completely removed, and high-purity purified NMP with high purity that can be reused in semiconductor processes can be obtained.

Although exemplary embodiments of the present invention have been described for illustrative purposes only, those skilled in the art will appreciate that various modifications are possible, without departing from the essential characteristics of the present invention. Therefore, the embodiments disclosed in this specification are intended to explain, not limit, the present invention, and the spirit and scope of the present invention are not limited by the above embodiments. The protection scope of the present invention should be construed according to the following claims, and all techniques within the scope equivalent thereto should be construed as being included in the scope of the present invention.

## Claims

1. A method for purifying waste N-methyl-2-pyrrolidone, the method comprising the steps of:
reacting the waste N-methyl-2-pyrrolidone containing an amine compound with an acid anhydride;
converting the amine compound into an amide compound; and
removing the amide compound by distillation.

2. The method for purifying waste N-methyl-2-pyrrolidone of claim 1, wherein a basic material is added in the step of converting into the amide compound.

3. The method for purifying waste N-methyl-2-pyrrolidone of claim 1 or 2, wherein an antioxidant is added in the distillation step.

4. The method for purifying waste N-methyl-2-pyrrolidone of any one of claims 1 to 3, wherein the weight of the acid anhydride is 2 times or more of the weight of the amine compound contained in the waste N-methyl-2-pyrrolidone.

5. The method for purifying waste N-methyl-2-pyrrolidone of any one of claims 1 to 4, wherein the amine compound is one or more selected from the group consisting of methylamine, dimethylamine, monomethanolamine, monoethanolamine, diethanol amine, 3-amino-1-propanol, 2-amino-1-propanol, methyl monoethanolamine, 4-amino-1-butanol, 5-amino-1-pentanol, ethyl monoethanol amine, propyl monoethanolamine, butyl monoethanolamine, 2-amino-2-methylpropanol, 2-amino-2-ethyl-1-propanol, 2-amino-2-methyl-1,3-propanediol, and piperazine.

6. The method for purifying waste N-methyl-2-pyrrolidone of any one of claims 1 to 5, wherein the acid anhydride is one or more selected from the group consisting of maleic anhydride, bromomaleic anhydride, phenylmaleic anhydride, succinic anhydride, methylsuccinic anhydride, phenylsuccinic anhydride, phthalic anhydride, 3-hydrophthalic anhydride, tetrahydrophthalic anhydride, 3-nitrophthalic anhydride, 3-fluorophthalic anhydride, hexahydrophthalic anhydride, 4-tert-butylphthalic anhydride, pyromellitic dianhydride, itaconic anhydride, citraconic anhydride, 3-methylglutaric anhydride, 3,3-tetramethyleneglutaric anhydride, 2-phenylglutaric anhydride and 1-cyclopentene-1,2-dicarboxylic anhydride.

7. The method for purifying waste N-methyl-2-pyrrolidone of any one of claims 1 to 6, wherein the amide compound is represented by the following structure: 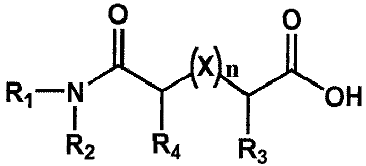 wherein:
n is an integer of 0 or 1,
X is a single or double bond when n is 0, and X is C(R')(R") when n is 1,
R₁ and R₂ are each independently selected from the group consisting of hydrogen, a C₁-C₂₀ alkyl group, a C₆-C₃₀ aryl group, a C₆-C₃₀ cycloalkyl group, a C₁-C₂₀ alkoxy group, a C₆-C₃₀ aryloxy group, -OH and a C₁-C₂₀ alkyl alcohol group, with the proviso that the case where both R₁ and R₂ are hydrogen is excluded, and R₁ and R₂ may be bonded to each other to form a ring,
R₃ and R₄ are each independently selected from the group consisting of hydrogen, halogen, a C₁-C₂₀ alkyl group, a C₂-C₂₀ alkenyl group, a C₆-C₃₀ aryl group, a C₆-C₃₀ cycloalkyl group, -OH and a C₁-C₂₀ alkyl alcohol group, and R₃ and R₄ may be linked to each other to form a ring, and the ring may be substituted with one or more R₅, wherein R₅s are the same or different from each other,
R' and R" are each independently selected from the group consisting of hydrogen, a C₁-C₂₀ alkyl group, a C₂-C₂₀ alkenyl group, a C₆-C₃₀ aryl group and a C₆-C₃₀ cycloalkyl group, and R' and R" may be linked to each other to form a ring,
R₅ is selected from the group consisting of hydrogen, halogen, nitro group, a C₁-C₂₀ alkyl group, a C₂-C₂₀ alkenyl group, a C₆-C₃₀ aryl group, a C₆-C₃₀ cycloalkyl group, -OH and a C₁-C₂₀ alkyl alcohol group, and
H in the alkyl group, the alkenyl group, the aryl group, the cycloalkyl group, the alkoxy group, the aryloxy group and the alkyl alcohol group may be replaced with a C₁-C₂₀ alkyl group, an alcohol group, or a C₁-C₂₀ alkyl alcohol group.

8. The method for purifying waste N-methyl-2-pyrrolidone of any one of claims 1 to 7, wherein the waste N-methyl-2-pyrrolidone containing the amine compound represented by Formula 1 reacts with the acid anhydride represented by Formula 2 to produce the amide compound represented by Formula 3, as shown in the following Reaction Scheme 1: 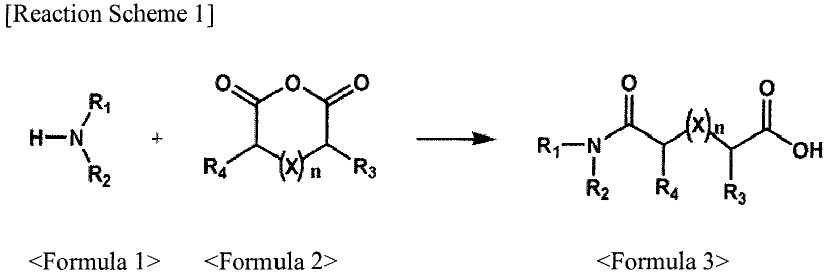 wherein:
n is an integer of 0 or 1,
X is a single or double bond when n is 0, and X is C(R')(R") when n is 1,
R₁ and R₂ are each independently selected from the group consisting of hydrogen, a C₁-C₂₀ alkyl group, a C₆-C₃₀ aryl group, a C₆-C₃₀ cycloalkyl group, a C₁-C₂₀ alkoxy group, a C₆-C₃₀ aryloxy group, -OH and a C₁-C₂₀ alkyl alcohol group, with the proviso that the case where both R₁ and R₂ are hydrogen is excluded, and R₁ and R₂ may be bonded to each other to form a ring,
R₃ and R₄ are each independently selected from the group consisting of hydrogen, halogen, a C₁-C₂₀ alkyl group, a C₂-C₂₀ alkenyl group, a C₆-C₃₀ aryl group, a C₆-C₃₀ cycloalkyl group, -OH and a C₁-C₂₀ alkyl alcohol group, and R₃ and R₄ may be linked to each other to form a ring, and the ring may be substituted with one or more R₅, wherein R₅s are the same or different from each other,
R' and R" are each independently selected from the group consisting of hydrogen, a C₁-C₂₀ alkyl group, a C₂-C₂₀ alkenyl group, a C₆-C₃₀ aryl group and a C₆-C₃₀ cycloalkyl group, and R' and R" may be linked to each other to form a ring,
R₅ is selected from the group consisting of hydrogen, halogen, nitro group, a C₁-C₂₀ alkyl group, a C₂-C₂₀ alkenyl group, a C₆-C₃₀ aryl group, a C₆-C₃₀ cycloalkyl group, -OH and a C₁-C₂₀ alkyl alcohol group, and
H in the alkyl group, the alkenyl group, the aryl group, the cycloalkyl group, the alkoxy group, the aryloxy group and the alkyl alcohol group may be replaced with a C₁-C₂₀ alkyl group, an alcohol group, or a C₁-C₂₀ alkyl alcohol group.

9. A method for purifying waste N-methyl-2-pyrrolidone, the method comprising the steps of
a first step of reacting waste N-methyl-2-pyrrolidone containing an amine compound represented by Formula 1 with an acid anhydride represented by Formula 2 to produce an amide compound represented by Formula 3, as shown in the following Reaction Scheme 1;
a second step of distilling waste N-methyl-2-pyrrolidone containing the amide compound; and
a third step of removing the amide compound having a higher boiling point than the waste N-methyl-2-pyrrolidone in the distillation step and obtaining purified N-methyl-2-pyrrolidone,
wherein in the first step, a basic material is further added to proceed with the reaction, and in the second step, an antioxidant is added: 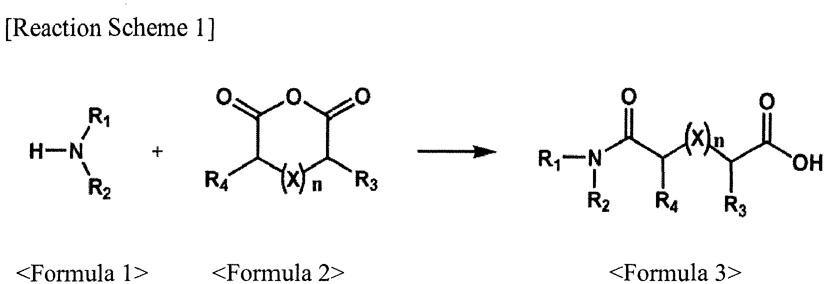
wherein:
n is an integer of 0 or 1,
X is a single or double bond when n is 0, and X is C(R')(R") when n is 1,
R₁ and R₂ are each independently selected from the group consisting of hydrogen, a C₁-C₂₀ alkyl group, a C₆-C₃₀ aryl group, a C₆-C₃₀ cycloalkyl group, a C₁-C₂₀ alkoxy group, a C₆-C₃₀ aryloxy group, -OH and a C₁-C₂₀ alkyl alcohol group, with the proviso that the case where both R₁ and R₂ are hydrogen is excluded, and R₁ and R₂ may be bonded to each other to form a ring,
R₃ and R₄ are each independently selected from the group consisting of hydrogen, halogen, a C₁-C₂₀ alkyl group, a C₂-C₂₀ alkenyl group, a C₆-C₃₀ aryl group, a C₆-C₃₀ cycloalkyl group, -OH and a C₁-C₂₀ alkyl alcohol group, and R₃ and R₄ may be linked to each other to form a ring, and the ring may be substituted with one or more R₅, wherein R₅s are the same or different from each other,
R' and R" are each independently selected from the group consisting of hydrogen, a C₁-C₂₀ alkyl group, a C₂-C₂₀ alkenyl group, a C₆-C₃₀ aryl group and a C₆-C₃₀ cycloalkyl group, and R' and R" may be linked to each other to form a ring,
R₅ is selected from the group consisting of hydrogen, halogen, nitro group, a C₁-C₂₀ alkyl group, a C₂-C₂₀ alkenyl group, a C₆-C₃₀ aryl group, a C₆-C₃₀ cycloalkyl group, -OH and a C₁-C₂₀ alkyl alcohol group, and
H in the alkyl group, the alkenyl group, the aryl group, the cycloalkyl group, the alkoxy group, the aryloxy group and the alkyl alcohol group may be replaced with a C₁-C₂₀ alkyl group, an alcohol group, or a C₁-C₂₀ alkyl alcohol group.
